Europäisches Patentamt

European Patent Office

Office européen des brevets

(11) Publication number: **0 487 994 A1**

# EUROPEAN PATENT APPLICATION

(21) Application number: **91119506.3**

(22) Date of filing: **15.11.91**

(51) Int. Cl.5: **A61L 2/18, G02C 13/00**

(30) Priority: **29.11.90 IT 2224290**

(43) Date of publication of application:
**03.06.92 Bulletin 92/23**

(84) Designated Contracting States:
**AT BE CH DE DK ES FR GB GR IT LI LU NL SE**

(71) Applicant: **CARL ZEISS S.p.A.**
**Via Caldera, 21/C3**
**I-20153 Milano(IT)**

(72) Inventor: **Bayer, Thomas**
**Via Caldera, 21/C3**
**I-20153 Milano(IT)**
Inventor: **Saccani, Renato**
**Via Caldera, 21/C3**
**I-20153 Milano(IT)**

(74) Representative: **Minoja, Fabrizio**
**Studio Consulenza Brevettuale Via Rossini, 8**
**I-20122 Milano(IT)**

(54) A method for the disinfection and cleansing of contact lenses.

(57) A method for disinfecting and cleansing contact lenses in a single operation which consists in immersing the contact lenses in a isotonic solution buffered with oxygen releasing compounds and the subsequent decomposition of said compounds with metal ions; said method allows the direct application of the lenses without previous washing operations.

EP 0 487 994 A1

The present invention relates to a method for the disinfection and cleansing of contact lenses by means of a single operation, which consists in immersing the contact lenses in an isotonic solution, which is buffered with oxygen releasing compounds, and subsequently decomposing said compounds with metal ions, with no need to wash the lenses before their application to eyes.

The well-known methods for the disinfection and cleansing of contact lenses, particularly for hydrophilic contact lenses, involve several drawbacks, due to the fact that the compounds used in such procedures can be absorbed by the polymers which form the lenses and then can be released by the same during the lens application, thus giving rise to eye irritation. A simpler method has been achieved using solutions which contain hydrogen peroxide in concentrations ranging from about 0.6 to about 3%; however, immersing the lenses in a neutralizing solution in order to eliminate residual hydrogen peroxide is always necessary. Therefore, drawbacks of various kind, such as casual contaminations, can occur. Moreover, in the neutralizing solution catalase is generally used, which can cause allergic effects in some subjects, as well as protein residues on the lenses.

Organochlorine derivatives capable of releasing free chlorine gradually have also been suggested. Although this system can be accomplished in a single operation, some drawbacks occur, since active chlorine easily reacts with the functional groups of the polymers used for the manufacture of the lenses, which are damaged after repeated disinfections (yellowing, physico-chemical alteration).

It has now been found a method for the disinfection and cleansing of contact lenses which allows to overcome the drawbacks related to the up to now known methods. The method of the present invention is characterized in that it can be performed in a single operation without need of washing or neutralizing operations.

The method of the invention consists in immersing the contact lenses in an isotonic solution, which is buffered with oxygen releasing compounds, and subsequently decomposing said compounds with metal ions.

The oxygen releasing compounds are selected from the group consisting of strong oxidizers, which release oxygen while contacting organic compounds, like mucins, fats, proteins or microorganisms, which can be present on contact lenses. Nascent oxygen, which is released from said oxidizers, reacts with the functional groups of organic compounds, causing the lysis of bacteria and macromolecules, which form the deposits to be eliminated from the contact lens surface.

Soluble inorganic or organic persalts, like potassium perchlorate, potassium or ammonium peroxydisulfate, potassium peroxymonosulfate, sodium peroxyborate, sodium peroxybenzoate, sodium peroxychlorobenzoate, optionally combined each other, can be used as oxidizing agents according to the present invention. The necessary amount so that said compounds perform a disinfecting and cleansing effect on lenses, in a period which is generally between 2 and 4 hours, normally ranges between 50 and 500 mg. At the end of disinfection and cleansing process, said compounds are decomposed by means of catalytic amounts of metal ions, particularly silver, manganese, cobalt, chromium or other transition metal ions.

According to a preferred embodiment, the invention provides for tablets, which can extemporarily be dissolved in an aqueous vehicle, containing the oxygen prompt-releasing compounds and microencapsulated metal ions, which are released only after a time necessary for the complete disinfection of contact lenses.

Obviously, said tablets can optionally contain conventional excipients and buffering agents, to provide a final ready-to-use solution having pH between 7 and 7.4.

The amounts of the various component for each tablet are not critical and are easily determined by those skilled in the art.

The amount of metal ions, which is necessary for the persalt catalytic decomposition will normally range from 50 to 800 $\mu$g, according to the amount of oxidizer and the kind of the selected ion. Whenever silver ion is used, remarkably lower amounts are needed, for example from 5 to 10 $\mu$g for each tablet. Catalyzing metal ions are provided by metal salts, which are microencapsulated with film forming agents, such as polyvinylpyrrolidone, hydroxyethylcellulose or other cellulose derivatives, which are normally used in microencapsulation techniques.

Adjustment in release delay of metal ions is achieved with known methods, suitably changing the mixture of film forming agents and the thickness of coating layer. The following examples further illustrate the invention.

**EXAMPLE 1**

A 162 mg tablet contains:

| Sodium peroxybenzoate | 50 mg |
|---|---|
| Silver acetate | 0,1 mg |
| Sodium acetate | 10 mg |
| Citric acid | 10 mg |
| Anhydrous sodium chloride | 70 mg |
| Polyvinylpyrrolidone | 20 mg |
| Cutin | 1 mg |
| Polyethylene glycol 6,000 | 2 mg |

Preparation for 100 tablets

A mixture containing 1 g of sodium acetate, 1 g of anhydrous citric acid, 10 mg of silver nitrate and 1 g of polyvinylpyrrolidone was kneaded with 1 ml of anhydrous ethyl alcohol, then was sifted through a 70 mesh sieve; the obtained granules were dried in nitrogen stream, then were transferred into a coating pan, where 10 ml of an alcoholic solution containing 1 g of polyvinylpyrrolidone and 100 mg of cutin were sprayed thereon.

The coated granules were mixed with 5 g of sodium peroxybenzoate, 7 g of anhydrous sodium chloride and 200 mg of polyethylene glycol 6,000. The mixture was tabletted in a suitable tabletting machine, thus obtaining tablets weighing about 162 mg.

One of these tablets was put in 10 ml of sterilized water wherein a lens to be disinfected and cleansed was immersed. After 2 hours, the lens was disinfected, and after 4 hours, peroxybenzoate resulted completely decomposed.

**EXAMPLE 2**

A 470 mg tablet contains:

| Sodium peroxyborate trihydrate | 275 mg |
|---|---|
| Potassium peroxydisulfate | 50 mg |
| Silver nitrate | 0,001 mg |
| Manganese sulfate | 0,60 mg |
| Anhydrous sodium chloride | 20 mg |
| Polyvinylpyrrolidone | 30 mg |
| Cutin | 0,1 mg |
| Polyethylene glycol 6,000 | 20 mg |

The preparation and the use of the above tablets were according to the procedure described in Example 1.

**EXAMPLE 3**

An about 420 mg tablet contains:

| Sodium peroxyborate trihydrate | 280 mg |
|---|---|
| Citric acid anhydrous | 70 mg |
| Anhydrous sodium chloride | 50 mg |
| Polyvinylpyrrolidone | 15 mg |
| Cobalt acetate tetrahydrate | 0,50 mg |
| Cutin | 0,1 mg |
| Hydroxyethylcellulose | 0,1 mg |

The preparation and the use of the above tablets were according to the procedure described in Example 1.

In vitro disinfecting activity

Disinfecting activity of an aqueous solution (10 ml), wherein a tablet of Example 3 was dissolved, was evaluated.

The following microorganisms were used: S. marcescens ATCC14756 or 14041, S. aureus ATCC6538; P. aeruginosa ATCC9027 or 15442, E. coli ATCC8739, C. albicans ATCC10231 and A. niger ATCC16404. 1 ml of the bacteria suspensions, which were previously diluted to $10^{-6}$, was placed into the containers, each containing 10 ml of sterilized water and a tablet of Example 3. After 4 hours, the disappearance of S. marcescens, S. aureus, P. aeruginosa and E. coli was observed, while C. albicans and A. niger resulted strongly inhibited.

Cleansing effect

30 Zeiss Krystal lenses were placed into 10 ml of a solution buffered at pH 7.2, containing 0.1% of lysozyme. The solution was heated to about 95°C for 30 minutes. Then, the lenses were removed, cooled and rinsed with distilled water. The deposit, which was formed on the lenses, was arbitrarily ranked 100 after observing it at the microscope at 100x. Then, the lenses were put into 10 ml of distilled water, wherein a tablet of Example 3 was immersed. After 4 hours, the lenses were removed and observed with a microscope 100 x, in order to evaluate the residual deposit. All the treated lenses resulted free from deposits.

**Claims**

1. A method for disinfecting and cleansing contact lenses in a single operation, which consists in immersing the contact lenses in a isotonic solution buffered with oxygen releasing compounds and the subsequent decomposition of said compounds with metal ions.

2. The method according to claim 1, characterized in that oxygen releasing compounds are selected from the group consisting of potassium perchlorate, potassium or ammonium peroxydisulfate, potassium peroxymonosulfate, sodium peroxyborate, sodium peroxybenzoate, sodium peroxychlorobenzoate.

3. The method according to claims 1 or 2, characterized in that metal ions are selected from the group consisting of chromium, cobalt, manganese, silver.

4. The method according to any one of the preceding claims, characterized in that the isotonic solution is obtained by extemporarily dissolving a tablet which promptly releases oxygen releasing compounds and subsequently releases the metal ions.

5. Tablets for the disinfection and cleansing of contact lenses to be extemporarily dissolved in an aqueous vehicle, said tablets containing oxygen promptly-releasing compounds and microencapsulated metal ions which will be released only after a time necessary to accomplish the complete disinfection and cleansing of contact lenses.

6. Tablets according to claim 5, characterized in that said ions are released after 2-4 hours.

7. Tablets according to claim 6, characterized in that said metal ions are microencapsulated with film forming agents selected from polyvinylpyrrolidone and hydroxyethylcellulose.

8. Tablets according to claims 5-7, containing from 50 to 500 mg of oxygen releasing compounds and from 5 to 800 $\mu$g of metal ions.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-2 003 033 (H.EVERS) | 1-4 | A61L2/18 |
| Y | * page 1, line 51 - line 62; claims * | 5-8 | G02C13/00 |
|  | * page 2, line 6 - line 11 * |  |  |
|  | --- |  |  |
| Y | EP-A-0 209 071 (TITMUS EUROCON KONTAKTLINSEN GMBH) | 5-8 |  |
|  | * page 4, line 4 - page 7, line 11 * |  |  |
|  | --- |  |  |
| X | EP-A-0 332 551 (RECHERCHE-CONCEPTION-DEVELOPMENT "R.C.D.LABO") |  |  |
|  | * claims * |  |  |
|  | --- |  |  |
| A | GB-A-2 173 017 (UNIVERSITY OF BATH) |  |  |
|  | --- |  |  |
| A | EP-A-0 227 987 (TANABE SEIYAKU CO.) | 5-8 |  |
|  | * page 5, line 18 - line 25 * |  |  |
|  | ----- |  |  |

TECHNICAL FIELDS SEARCHED (Int. Cl.5)

A61L
A01N

The present search report has been drawn up for all claims

| Place of search | Date of completion of the search | Examiner |
|---|---|---|
| THE HAGUE | 09 MARCH 1992 | G COUSINS-VAN STEEN |

EPO FORM 1503 03.82 (P0401)